# EUROPEAN PATENT APPLICATION

(11) **EP 4 122 529 A1**
(43) Date of publication of application: **25.01.2023**
(21) Application number: 21186483.0
(22) Date of filing: 19.07.2021
(51) Int. Cl.: A61N 1/36, A61N 1/04

(54) **COMPUTER-IMPLEMENTED METHOD FOR ENABLING PATIENT-SPECIFIC ELECTROSTIMULATION OF NEURONAL TISSUE AND ASSOCIATED DEVICES AND SOFTWARE**

(71) Applicant: Bottneuro AG, 4057 Basel (CH)
(72) Inventor: Osmani, Bekim, 4056 Basel (CH); Joodaki, Mahyar, 4054 Basel (CH)
(74) Representative: Mertzlufft-Paufler, Cornelius

(57) **Abstract**

For improving the quality of electrostimulation therapy, a computer-implemented method is proposed that allows patient-specific choice and/or configuration of an electrical stimulation device 1 (Fig. 2) that is designed and usable for electrostimulation therapy of neuronal tissue, in particular in the brain. The method benefits from pre-existing images 3, for example acquired using a medical imaging technique such as MRT or PET. Based on data extracted from such an image 3, a computer simulates possible electrical field distributions E(x,y,z), which would be achievable if the device 1 would be configured with a certain set of configuration parameters. By varying these parameters and repeating the simulations, the method allows optimization of the configuration parameters for a given design of the device 1. As a result, the method delivers a set of optimized configuration parameters, which, if applied to the device 1, allow a "best-case" electrostimulation with the device 1 that is tailor-made for the need of the patient (cf. Figure 1).

## Description

The present disclosure is related to the field of electrical stimulation of neuronal tissue for treatment of neurodegenerative diseases, in particular treatment of Alzheimer's disease (AD).

In particular, the invention concerns a computer-implemented method for computing a patient-specific set of configuration parameters suitable for configuring and/or choosing an electrical stimulation device that is designed for electrical stimulation and/or recording of neuronal tissue, in particular of cerebral tissue. Using this method, a patient-specific electrical stimulation of the patient's neuronal tissue is possible by choosing and or configuring the stimulation device according to the derived patient-specific set of configuration parameters. The invention also concerns a method for computing a series of individual sets of such optimized configuration parameters.

Furthermore, the invention concerns an associated device which is designed for this purpose and features a number of individually addressable non-invasive stimulation electrodes.

The invention further concerns a set of such devices, which are designed differently to fit the respective needs of a particular type of electrostimulation to be performed.

Finally, the invention also concerns associated software and hardware that enables the computer-implementation of the method mentioned above. Hence, a computer program and a computer-readable data carrier may be employed for implementing the method, as well as a system for patient-specific electrostimulation and also a computer system for patient-specific configuration of an electrical stimulation device.

It is important to note here that the method presented herein neither includes a step of treating the human or animal body by surgery or therapy and also no diagnostic step practiced on the human or animal body; rather the invention is concerned with benefiting from pre-existing diagnostic data sets (e.g. PET-images) and using them for configuring an electrical stimulation device in a "best-case" effort. After the method according to the invention has been performed, the configured device may be used by any person wishing to use it for efficient electro-therapy. It is also to be stressed, that the methods does not produce electrical field distributions in reality but rather virtually within a computer simulation. This avoids time-consuming and costly trial-and-error manual configuration of the stimulation device. Instead, the configuration can be fully computer-implemented and standardized, without significant intervention from a practitioner.

The method presented herein is particularly relevant to the treatment of Alzheimer's disease (AD), which is the 6th leading cause of death in the United States, with more than 5 million people affected by AD in the United States alone. Although the causes of AD are yet to be fully understood, this neurodegenerative disease is largely associated with amyloid plaques, neurofibrillary tangles, and loss of neuronal connections in the brain. AD results in a loss of cognitive, emotional and social abilities of the patient and causes immense costs within the health-care-system, with a strong growth in recent years, due to the aging populations in developed countries.

Amyloid plaques are extracellular deposits of the amyloid beta (Aβ) protein mainly in the grey matter of the brain, and are very often referred to as "debris". Current treatments of AD focus on clearing the brain from these plaques or debris. However, latest clinical studies have shed severe doubts on the effectiveness of this therapeutic approach.

Recently, it has been found that 40 Hz acoustic stimulation decreases amyloid beta deposition in the brain in certain patients (cf. Lee, J. et al: "40 Hz acoustic stimulation decreases amyloid beta and modulates brain rhythms in a mouse model of Alzheimer's disease" bioRxiv 2018).

Another important approach for treating AD is the electrical stimulation of glial cells (or neuroglia), in particular of microglia, which are non-neuronal cells in the central nervous system (brain and spinal cord) and the peripheral nervous system that - different from neurons - do not produce electrical impulses. Microglia are a type of neuroglia (glial cells) located throughout the brain and spinal cord. Microglia are resident macrophage cells, which act as the first and main form of active immune defense in the central nervous system and have been found to be highly important to the development of AD. Neurons (or nerve cells), on the other hand are electrically excitable cells that communicate with other cells via specialized electro-chemical connections called synapses. Neurons are the main component of the nervous tissue, in particular in the brain.

This approach is sometimes referred to as "low intensity transcranial electric stimulation", because the electrical fields are typically produced outside of the brain using non-invasive stimulation electrodes and thus transferred into the brain via the skull bones (thus "transcranial"). It has not only been applied to the treatment of AD but also to mental diseases such as major depression or for treating neuropathic pain as a result of nerve injuries or tinnitus, Parkinson's disease, post-stroke aphasia. By modulating axonal membrane potential, transcranial current stimulation (tCS) can interact with various endogenous neural network features, such as ion channel dynamics, spike timing, firing rate, oscillatory potential, synaptic transmission, and also brain responses to external stimuli.

A typical DC-stimulation scheme employs a bipolar montage, in which there is at least one negatively charged cathode and at least one positively charged anode; however, AC-stimulation schemes have also been applied in the past.

Current methods are, however, limited in their effectiveness, because tCS typically results in an insufficient focusing of the currents injected / the electrical fields produced in the brain. Therefore, multifocal tCS has been developed using a number of non-invasive stimulation electrodes. It has also been found, that the duration of electrostimulation, both on large and small time-scale, and also the duration and repetition of an individual stimulation session play important roles for the success of the therapy.

Furthermore, gamma frequency stimulation, which is a specific type of electrostimulation in a frequency range of between 35..100 Hz, has been proposed for treatment of AD (cf. Martorell, A.J. et al. "Multi-sensory Gamma Stimulation Ameliorates Alzheimer's-Associated Pathology and Improves Cognition". Cell (2019))

In this context, one objective of the invention is to provide means which allow patient-specific and hence effective electrostimulation of neuronal tissue, in particular in the brain. A particular desire is that the means should enable a largely autonomous therapy by electrostimulation that can be performed by the patient himself, remote from a hospital. The approach of the invention, however, is not only applicable to the treatment of neurodegenerative diseases such as AD but also to other sorts of electrostimulation of neuronal tissue, also beyond therapeutic aims.

In accordance with the present invention, a method is provided according to claim 1, which solves the afore-mentioned problem. In particular the invention proposes an (at least partly) computer-implemented method as introduced at the beginning, which, in addition, may be characterized in that the method comprises the following steps:
A) extracting patient-specific data from at least one pre-existing image (3) previously acquired using a medical imaging technique;
B) computer simulation of an electrical field distribution that can be generated using the electrical stimulation device (1) when configured with a specific set of configuration parameters, taking into consideration the extracted patient-specific data;
C) Variation of a set of simulation parameters during iterative application of step B) to compute the patient-specific set of configuration parameters, which are thus optimized for the patient.

In other words, the method may be implemented by:
producing a numerical and/or geometrical model of the patients neuronal tissue to be electro-stimulated;
performing computational modeling of electrostimulations possible with the device after it has been configured and/or chosen according to a specific set of configuration parameters;
and finally identification of an optimized set of these configuration parameters (which may include the choice of a particular stimulation device comprised in a limited set of M available stimulation devices), which - if applied to the chosen / present stimulation device, will lead to a patient-specific electrostimulation that is optimized for the needs of the patient.

A bit simplified, the major aim of the method is to derive the configuration parameters necessary for achieving an electrostimulation with the stimulation device that represents a best-case scenario, given typical boundary conditions such as the available number of stimulation electrodes, their physical shape and/or geometrical arrangement as well as other limiting factors such as current and frequency range available for the driving currents to be applied to the individual electrodes.

Most conveniently, the simulation parameters and/or the final set of configuration parameters may be processed as a digital data set, respectively. In addition, the simulation parameters may comprise (in some cases, they may be identical to) the configuration parameters.

Step B may be performed according to an optimization method, in particular including a FEM-method for calculating the E-field distributions. The goal of the optimization can be maximizing an electrical field strength in at least one region of interest, in particular while minimizing the electrical field strength elsewhere.

The variation of the simulation parameters will typically comprise a variation of said configuration parameters, for example (virtually) moving a location of one of the N electrical stimulation electrodes or (virtually) adapting an 3D-arrangement of the N stimulation electrodes of the device.

To provide one possible application case, it is well known, that the activity of glial cells can be modulated by electrical stimulation. In response to electrical fields of a well-defined field strength and frequency, these cells can be stimulated to secrete anti-inflammatory agents (e.g. IGF1, TGFB, IL-10). One particularly suitable approach is the electrical stimulation to inhibit the release of pro-inflammatory cytokines and to promote the release of anti-inflammatory cytokines und insulin degrading enzymes (IDE). By this approach, the glial cells can be transferred from a so-called M1 state, in which there is an abundance of pro-inflammatory cytokines, which are prominent in AD, to a so-called M2 state, in which there is an surplus of anti-inflammatory, debris clearing cytokines.

In the brain tissue, gray matter is distinguished from white matter: gray matter is a major component of the central nervous system, consisting of neuronal cell bodies, neuropil (dendrites and unmyelinated axons), glial cells (astrocytes and oligodendrocytes), synapses, and capillaries. Gray matter is distinguished from white matter in that it contains numerous cell bodies and relatively few myelinated axons, while white matter contains relatively few cell bodies and is composed chiefly of long-range myelinated axons. The color difference arises mainly from the whiteness of the myelin.

The proposed extraction of patient-specific data during step A) of the method can include such information, in particular the distribution of gray and white matter within the brain, and thereby also a local distribution of glial cells. Hence, such patient-specific data allows the (preferably computer-implemented) identification of at least one, i.e. in particular several, region(s) of interest (ROI) inside the brain to be electro-stimulated. The task to be solved during steps B) and C) of the method is thus to derive the set of configuration parameters such that a focused, i.e. localized, electrostimulation of the identified at least one ROI is possible, after the electrical stimulation device has been configured with or at least chosen (from a set of available devices) based on said optimized configuration parameters.

The patient-specific data extracted, preferably by a computer implemented algorithm, from the at least one image, can also be fed to a computational module, which computes a patient-specific head/skull model from that data. Such a head model can thus form the basis of the computer simulations to be performed during step B).

The computational modeling / simulation performed in step B) aims at computing a number of electrical field distributions, which - in principal - could be generated, for example using either a fixed set of N stimulation electrodes that can be freely arranged (i.e. using a stimulation device with a configurable electrode arrangement) or a given set of N stimulation electrodes arranged in a fixed (or only slightly adaptable) arrangement.

As explained previously, the method may aim at enabling focused electrostimulation in at least one identified region of interest (ROI). For this purpose, it is important not to stimulate tissue regions outside of this ROI. This may be achieved by configuring the stimulation device such that regions outside of the ROI only receive low magnitudes of the electrical field (and resulting injected currents) generated with the device. For enabling a best-possible focused electrostimulation, a sub-critical electrostimulation of the regions out of the ROI is proposed. "Sub-critical electrostimulation" may be achieved, by making provisions that the electrical fields remain below an action potential (AP) of neuronal cells present in the ROI. For example, the AP of typical neuronal cells in the brain is in the order of 50 mV. Taking into account the typical size of a neuron of 20 to 30 µm, this results in an upper limit for the electrical field strength to be applied in the order of 2.0 kV/m. Therefore, the computer simulation may be directed at safeguarding such "sub-critical electrostimulations" in tissues regions outside the ROI.

The method will now be described in greater detail: For example, step C) of the method may comprise a variation of a physical configuration of N stimulation electrodes of the stimulation device. In particular, the variation may comprise a change of a location of one of the N stimulation electrodes or a size and/or shape of one of the N stimulation electrodes.

It is also understood, that N stimulation electrodes of the stimulation device may be used later on during therapy to generate one optimized electrical field distribution computed in the simulation. Hence the electrical field distribution can be generated with the N stimulation electrodes.

The set of patient-specific configuration parameters derived with the method may comprise at least one, preferably at least two, of the following parameters:
a specific geometrical arrangement of N stimulation electrodes of the stimulation device achievable by re-arranging the electrodes within a limited geometrical adjustment range offered by the stimulation device (for example, the stimulation device may be designed as a head bonnet with the stimulation electrodes movable on the bonnet within a limited xy-adjustment range);
a specific geometrical arrangement of N stimulation electrodes belonging to one particular stimulation device comprised in a limited number M of stimulation devices each featuring a pre-defined, in particular non-reconfigurable, respective arrangement of the N stimulation electrodes (Hence the result of the optimization may be a choice of one particular of the M stimulation devices);
a relative distribution of N driving currents or driving voltages to be applied to N stimulation electrodes of the stimulation device (hence a certain electrical driving scheme may be part of the configuration parameters);

The derived configuration parameters can also comprise a patient-specific electrical stimulation protocol for driving a number of N stimulation electrodes of the device. In such a case, the stimulation protocol may comprise at least one, preferably at least two, of the following electrical stimulation parameters:
- amperage and/or
- frequency and/or
- ac/dc driving scheme and/or
- relative phase
of individual driving currents to be applied to the individual stimulation electrodes of the stimulation device.

In addition, the method may further comprise a step of
D) configuring (either manually or computer-assisted or fully computer-implemented) the stimulation device with the derived set of configuration parameters, in particular with said electrical stimulation protocol.

In particular, the configuration of the stimulation device may be performed such that the stimulation device is configured to focus the electrostimulation onto at least one region of interest (ROI) that has been extracted from the at least one image during step A).

In other words, the stimulation device may be configured and/or chosen (in particular from a limited set of pre-configured stimulation devices, as described below) according to the set of configuration parameters, which was optimized during application of steps B) and C).

Furthermore, the method may also comprise (alternatively or additionally) a step of
E) choosing said electrical stimulation device out of a pre-defined set of M different stimulation devices based on the derived set of configuration parameters.

Following this approach, the M stimulation devices comprised in the pre-defined set may differ:
in their respective number N of available individual stimulation electrodes; and/or
in a respective, fixed or adjustable, geometrical arrangement of a number of N stimulation electrodes; and/or
in a respective individual shape of at least one of the respective stimulation electrodes.

In other words, each of said M stimulation devices may be pre-configured for producing a certain electrical field distribution.

For efficient therapy later on with the device, it is also beneficial if the patient-specific data extracted in step A) comprise at least one, preferably ate least two, of the following patient-specific parameters:
geometrical data of a skull of the patient, such as distribution of a thickness of the skull bone and/or of the scalp - such data are highly useful for computing an accurate head model, which can form the basis of the desired computer simulation of the electrical field distributions;
3D-distribution of brain tissue, in particular size of the patient's brain and/or distribution of gray and white matter within the brain of the patient - such parameters form important boundary conditions for the computer simulation;
localized concentrations of specific proteins, in particular of amyloid and/or tau proteins, within the brain of the patient - such data can help to focus the electrical fields generated to ROI;
localized occurrence of atrophy in the brain of the patient - such data can be beneficial for fine-tuning the focus of the electrical fields to be generated;
3d-coordinates of at least one region of interest (ROI) to be electro-stimulated with the device (1);

In all of such cases, it may be preferable if the optimization performed in step B) takes into account at least one of the these patient-specific parameters for computing said (optimized) set of configuration parameters, because this will help in configuring the stimulation device according to the specific needs of a particular patient.

Likewise, it will be beneficial, if the optimization performed in step B) is based on a geometrical head model derived from the patient-specific data extracted in step A), because this allows more accurate computer simulations.

The simulation parameters varied in step C) may comprise at least one, preferably at least two, of the following parameters:
number N of available stimulation electrodes (2) of the device;
geometrical arrangement of N available stimulation electrodes of the device;
respective shape of the available stimulation electrodes of the device
amperage and/or frequency of driving currents to be applied to individual stimulation electrodes of the device;
electrical ac/dc-driving scheme to be applied to the device;

In addition, step C) may be performed
to maximize a local electrical field strength in at least one region of interest (ROI) extracted from the at least one image in step A), in particular while minimizing the electrical field strength in remaining tissue regions; and/or
using a state-of-the-art optimization algorithm, preferably based on a finite-element-method;
By such approaches, it can be safeguarded that in remaining regions outside of the at least one region of interest (ROI), extracted from the at least one image in step A), an effective field strength remains below a physiological action potential (AP) of neuronal cells present in these remaining regions ("sub-critical stimulation" c.f. above).

As examples, during step C) at least one, preferably at least two, of the following boundary conditions may be taken into account:
maximum driving voltage and/or driving current available for driving an individual stimulation electrode of the device;
available frequency range for a driving current to be applied to an individual stimulation electrode of the device;
number M of available fixed geometrical arrangements of N stimulation electrodes provided by a limited set of M available different stimulation devices;
maximum number Nₘₐₓ of available stimulation electrodes;
limited number W of different available shapes of stimulation electrodes.

The pre-existing images from which the computer extracts the patient-specific data may be present as a digital data set. For achieving an efficient and meaningful optimization, the at least one pre-existing image may comprise at least one of, preferably at least two of,
a magnetic resonance tomography (MRT) image (3) and/or
a positron emission tomography (PET) image (3) and/or
a X-ray computed tomography (CT) image (3).

A highly useful approach, in particular for efficiently therapy of AD with the device later on, is implemented, if step A) comprises extracting localized concentrations of particular proteins (which define respective regions of interest (ROI)) from pre-existing PET-images. In this case, the PET-images may have been acquired (previously, before performing the method) using protein-specific, in particular radio-active, markers. Such state-of-the-art clinical imaging techniques allow identification of a ROI with high specificity and high spatial resolution, which are both important for the later success of the electrostimulation therapy (which is not part of the method presented herein, however).

The stimulation device can also feature at least one electrode (in particular one of said N stimulation electrodes) configured or (at least) configurable (e.g. electronically) for recording electrical fields generated with some of the (remaining) N stimulation electrodes of the device and/or for recording nerve signals from neuronal tissue, in particular brain waves (which are normally recorded using an electroencephalography (EEG)-device). Such a design has the advantage, for example, that during therapy, the stimulation device can stimulate the brain for a first period of time, followed by recording of brain waves for a second period of time, and so on.

Measurement data obtained with this at least one recording electrode may be used as another valuable input for optimizing the configuration parameters during step B) and C). In other words, electrical fields and or nerve signals, which may have been previously recorded with the stimulation device, can be used as an input to be considered during step B).

For example, an electrical field produced by a subset of the N stimulation electrodes may be read-out with at least one electrode of the device, which is (momentarily or permanently) configured as a recording electrode. This read-out may then serve to rearrange at least one of the N stimulation electrodes (as a possible result of the optimization performed during step B) and C).

This approach may be also be done iteratively, such that a loop of stimulation & recording, optimization, followed by repetition of stimulation & recording and subsequent optimization may be achieved. Such a loop can be highly beneficial for achieving optimized electrical stimulation of deep brain regions, in particular taking into account resonance effects (i.e. a synchronization with brain waves, which can be detected by a set of recording electrodes) occurring during stimulation.

For solving the afore-mentioned problem, there is also proposed a method for computing a series of individual sets of optimized configuration parameters. Each of these different sets is computed by performing a method according to one of the claims directed towards a computer-implemented method for for computing a patient-specific set of configuration parameters (to be applied to an electrical stimulation device) or as described before.

Each of the individual sets may be intended for configuring the stimulation device prior to a specific therapy session of a series of such sessions. This way, each of the sets can be optimized for enabling a pre-defined electrostimulation-pattern to be generated with the stimulation device during a particular session. Advantageously, the different consecutive electrostimulation-patterns may define a specific electro-therapy scheme that is to be applied to the patient in a series of electro-therapy sessions.

The sets of configuration parameters may differ in at least one of the configuration parameters, for example in an arrangement / location of individual stimulation electrodes and/or in a parameter of an electrical stimulation protocol applied to the stimulation device.

Following this inventive concept, the afore-mentioned problem is solved by an electrical stimulation device, as defined by the claims. Thus, the invention also proposes an electrical stimulation device as introduced at the beginning, which, in addition, is characterized in that the device comprises a number of N non-invasive stimulation electrodes arranged in a 3D-geometry, an electronic driving circuit for providing individual electrical driving voltages or driving currents to the individual stimulation electrodes, and a data interface for configuring said stimulation device with a patient-specific set of configuration parameters.

The electrodes can be of a round or angular shape; they can also be designed as a wire or strip. When using strips, for example, the electrodes may be customized to the head of a patient. They can also be embedded in a cloth or mesh of the stimulation device. Such designs are beneficial for efficiently stimulating specific areas of the head, for example.

For such a device, it is preferable if the stimulation device is then also configured with a set of configuration parameters that have been derived with a method according to one of the previous claims, because this allows a "best-use" of the device.

The 3D-geometry of the stimulation electrodes may be fixed or slightly re-configurable within a certain geometrical adjustment range, depending on the application.

A particular handy design of the stimulation device is as a bonnet to be worn by a patient on his skull. This bonnet may then carry the N stimulation electrodes, and preferably the driving circuit, and most preferably an electrical power supply for autonomous operation of the driving circuit. The bonnet may be tailored to the head of a patient, based on pre-existing medical images (MRI/CRT/PET) of the head.

Moreover, for reasons of patient safety, it is preferably if electrical interconnections between the stimulation electrodes and the driving circuit are embedded in the bonnet, in particular such that they cannot be manipulated by a patient without destructing the bonnet.

Such a stimulation device may be pre-configured in various ways. For example, a geometrical arrangement of the N stimulation electrodes of the device may be re-configurable, i.e. the electrodes may be movable in the respective 3D-location on the bonnet and/or they may be changeable in their shape (e.g. by replacing a respective electrode head).

The geometrical arrangement and/or shape of the N stimulation electrodes can also be permanently or initially fixed and hence not be re-configurable.

The, preferable wireless, data interface, on the other hand, may be configured to enable configuration and/or control of the stimulation device via an external, preferably mobile, electronic device, such as a computer or a tablet. That is, the data interface can provide a (preferably wireless and/or bidirectional) data link between the stimulation device and the additional electronic device, such that the stimulation device may be configured with the help of (or even automatically by) the electronic device.

As previously contemplated, the method may also take into account a set of pre-existing different electrical stimulation devices. The afore-mentioned problem is therefore also solved by a set of a limited number M (preferably 2<M<64) of electrical stimulation device, with each device offering a respective number of N individually addressable non-invasive stimulation electrodes. In particular, each of these M stimulation devices may be a stimulation device as described above or as defined in the claims. For solving the problem, the set is characterized in that the stimulation devices comprised in the set differ in terms of their respective number N of available individual stimulation electrodes; and/or in a respective fixed geometrical arrangement of a number of N stimulation electrodes; and/or in a respective individual shape of at least one of the respective stimulation electrodes.

Most preferably, the number M may be kept smaller than 10, such that the fabrication effort for providing the set is limited.

In addition, the afore-mentioned problem is solved by a system for patient-specific electrostimulation of neuronal tissue. For solving the problem this system comprises an electrical stimulation device, in particular according to one of the claims directed towards such a device or as described above; a, preferably mobile, electronic device, which can communicate with and control the electrical stimulation device via a, preferably wireless, data interface of the stimulation device.

The system is further characterized in that the electronic device is configured to transmit a set of configuration parameters to the stimulation device via the data interface of the stimulation device; understandably, these parameters may be derived using a method according to the invention.

According to a specific embodiment, the configuration parameters, in particular an electrical stimulation protocol, to be transmitted to the stimulation device prior to a (e.g. daily) therapy session (e.g. of a length of 30 min), may be adapted over time, following a certain electro-therapy scheme. Such adaptions may also be simulated and optimized, preferably prior to a series of therapy sessions to be performed, each time using a computer-implemented method according to the invention, as described previously. This approach allows definition of specific respective sets of defined configuration parameters for each therapy session of a series of therapy sessions.

Alternatively, or additionally, the electronic device may feature an internet interface for enabling remote-control of the electronic device and thereby of the stimulation device. By this approach, an electrostimulation that is later performed with the stimulation device can be live-monitored and/or stopped via a remote-connection to the electronic device, which has been established using its internet interface.

The internet interface may be implemented, for example, using a standard WLAN- or LAN-module built-into the electronic device.

Generally speaking, the invention also proposes a computer system for patient-specific configuration of an electrical stimulation device, which is designed for electrical stimulation and/or recording of neuronal tissue, in particular of cerebral tissue, for solving the afore-mentioned task. This system may comprise a typical processer, a data storage device coupled to the processor, and optionally a display device. The data storage device stores instructions (in particular in the form of a computer program as specified further below) operable to cause the processor to perform a method according to one of the claims directed towards a method or as described herein.

When the processor operates the instructions, the processor may extract patient-specific data from a pre-existing image (this image may also be present on the data storage device or accessible via a network interface operable by the processor, for example); the processor may then perform a simulation of an electrical field distribution that can be generated with the device when configured with a specific set of configuration parameters (the simulation may start with a pre-defined standard setting of these configuration parameters); this simulation can be performed repeatedly by the processor, each time varying a set of simulation parameters, i.e. in particular varying one of said configuration parameters; and finally the processor may output a final patient-specific set of configuration parameters, which are the result of an optimization performed during iterative repetition of said computer simulation.

The set of configuration parameters now finally optimized for the patient, may then be used to configure and/or choose the stimulation device, which can thus be made ready for performing a patient-specific electrical stimulation with the device (this step of treatment of the patient may thus follow the step of configuring the stimulation device).

There is also provided a computer program, which solves the afore-mentioned problem. This program, which may preferably be designed as an app to be run on a mobile electronic device such as a table or smart phone, comprises instructions, which, when the program is executed by a computer, in particular by said electronic device, cause the computer / said electronic device to carry out a method as laid out herein or as defined in the claims.

For achieving a patient-friendly solution, another computer program is proposed, which can also be designed as mobile app. This second program comprises instructions, which, when carried out by an external mobile electronic device or a computer, cause the computer / electronic device to communicate with an electrical stimulation device (which may be designed / configured as described herein or by the claims) and to configure the stimulation device with a set of configuration parameters that enable patient-specific electrostimulation. Of course, here again, it is most preferable, if the set of configuration parameters has been derived with a method as described herein or as defined by the claims.

Alternatively, or additionally, the instructions can also cause the computer / electronic device to establish a remote-connection to the electronic device via the internet. This way, the computer program may enable a live-monitoring of and/or stopping of an electrostimulation to be performed with the electrical stimulation device.

Such a Live-monitoring may comprise a monitoring of voltages or driving currents applied to the stimulation electrodes of the electrical stimulation device. In case these voltages or currents exceed maximum values defined to guarantee safe electrostimulation, a monitoring algorithm may automatically stop the performed electrostimulation via the remote-connection and also provide a warning to the user via the electronic device (acoustically and/or optically, e.g. by a banner on the display or the like).

The computer programs mentioned before may be sophisticated further to achieve further benefits for the patient: For example, the program can also comprise instructions, which, if carried out, by the electronic device cause the electronic device to offer the user a cognitive test. This offer may be generated by the electronic device before and/or after an electrostimulation has been performed with the electrical stimulation device. The offer can be presented to the patient in particular using a display and/or loudspeakers; answers form the patient may be recorded using a microphone of the electronic device and/or a touch display.

In addition, the patient can wear the stimulation device while performing such a cognitive test. This has the advantage that recording electrodes of the stimulation device (this may be stimulation electrodes, simply electronically reconfigured as recording electrodes) may be used to record neural signals of the patient during the cognitive test; these signals may be recorded by the electronic device as a bio marker. Hence, the instructions can cause the electronic device to record nerve signals (in particular brain waves) from the patient using a recording electrode of the stimulation device, while the patient performs the test.

This approach allows to digitally document a bio marker, for example a success rate of a cognitive test result, based on a response to that cognitive test, which the user has input into the electronic device via a user interface. In particular, the electronic device may record a time stamp and or parameters of a performed electrostimulation together with the bio marker. This way a highly informative bio marker data set can be formed, which can provide important information to HCP for steering the therapy in the future.

Last but not least, for solving the afore-mentioned problem, a computer-readable data carrier may be used (in particular for tangibly storing one of said computer programs) comprising instructions, which when executed by a computer, cause the computer to carry out a method according to one of the claims directed towards a method and/or as described herein. The digital output, resulting from carrying out the instructions, will thus be the desired set of configuration parameters, which are optimized for the individual patient and can be used to configure the electrical stimulation device. As a result, the device can provide a patient-specific electrical stimulation that is tailor-made for treating neuronal tissue of a particular patient.

It is obvious, that similar data carriers may be used which comprise instructions defining a computer program as described before or as defined in the claims.

Preferred examples of the present invention shall now be described in more detail, although the present invention is not limited to these examples.

With reference to the accompanying drawings:
- Fig. 1: is a schematic flow diagram, illustrating the individual steps of the method according to the invention,
- Fig. 2: illustrates a system for patient-specific electrostimulation of brain tissue, and
- Fig. 3: illustrates patient-specific configuration of an electrical stimulation device, as embedded in a more general concept of providing patient-specific diagnostic, care and therapy.

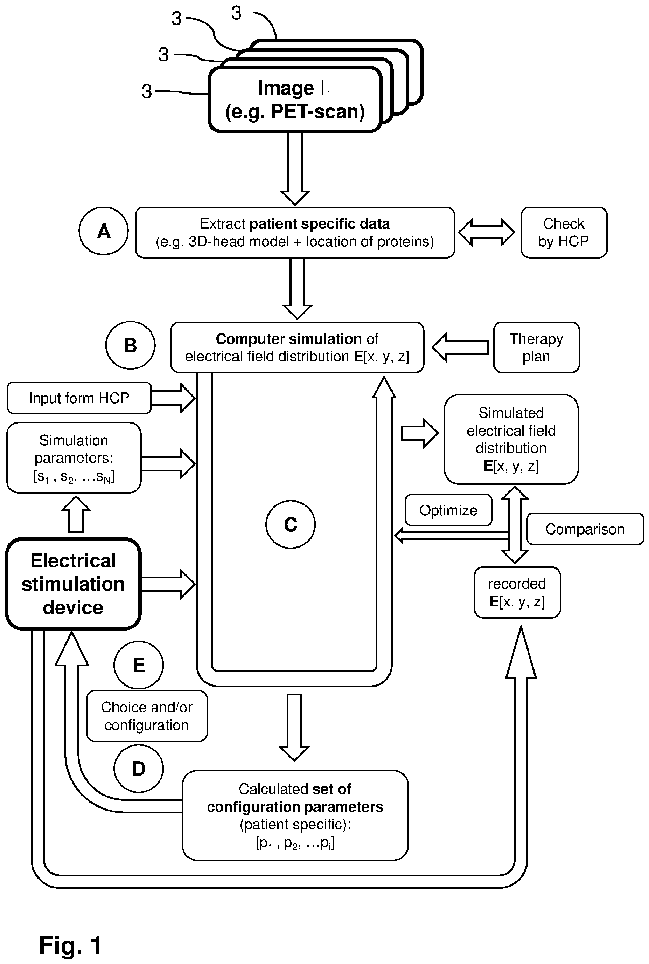

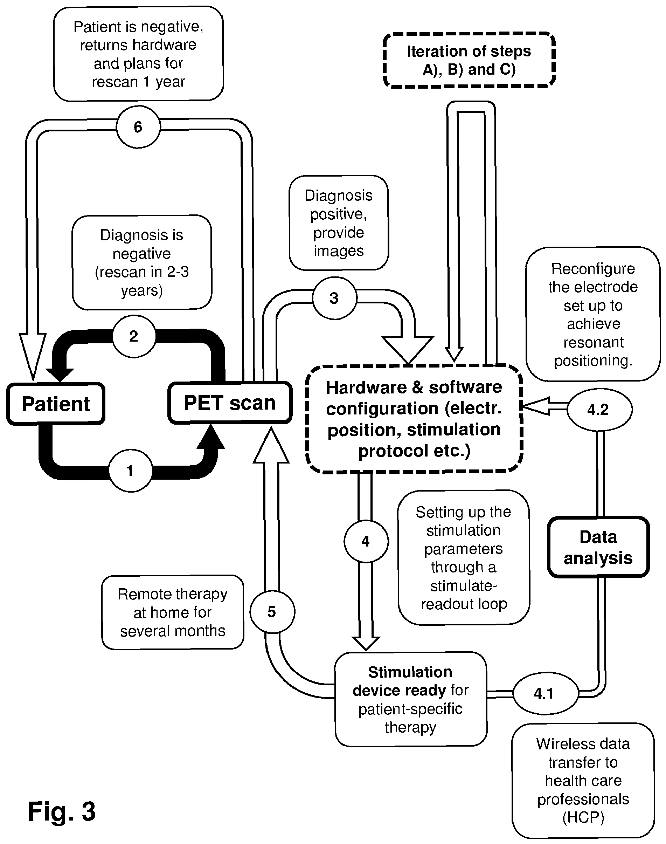

Figure 1 illustrates a computer-implemented method for deriving a patient-specific set of configuration parameters that can be used for configuring and/or choosing an electrical stimulation device 1, as the one shown in Figure 3, that is designed for electrical stimulation and/or recording of cerebral tissue.

As previously explained, the method may benefit from pre-existing images 3, e.g. MRT- or PET-scans, obtained from the patients head (as one example) in the hospital, where the necessary imaging equipment is available. The method may then be implemented on any suitable computer system, which may be remote from the hospital, even on another continent. Afterwards, health care professionals (HCP) or the patient himself may be provided with the derived set of optimized configuration parameters and the HCP or the patient himself (in particular remotely assisted via the internet, as will be explained below) may configure the stimulation device 1. After this step, the patient himself may perform an electrostimulation therapy safely himself, in particular at home, using the so-configured device 2.

As shown exemplarily in Figure 1, the method may benefit from a set of pre-existing images 3, which were acquired in a positron emission tomography (PET) scan of the skull of the patient. In a first step A), a processor of a computer system (that executes instructions defining a method as now explained) extracts a number of patient-specific data from the series of images 3, including a 3D-distribution of gray and white matter within the brain of the patient but also localized concentrations of specific proteins, namely amyloid and tau proteins. Based on this data, the processor defines several regions of interests (ROI) to be electro-stimulated.

These regions are presented by the computer system on a display such that a health care professional (HCP) can check and affirm them.

In addition, the processor may process further images 3, for example a series of X-ray computed tomography (CT) or magnetic resonance tomography (MRT) images 3 to extract geometrical data of the skull of the patient, such as distribution of a thickness of the skull bone and of the scalp. From this data, the processor can generate an accurate 3D-model (head model) of the skull that allows precise simulation results.

In a next step B), the computer simulates an electrical field distribution that would be achievable with one particular stimulation device 1, when configured with a specific set of start parameters. These start parameters may define a standard location of a number of N stimulation electrodes, their specific shape but also electrical parameters such as a current strength (amperage), frequency of a certain drive voltage applied to one of the stimulation electrodes (in case of AC-electrostimulation) or relative strengths of currents as distributed among the N electrodes.

Using this start set of simulation parameters, which can also include boundary conditions such as an available maximum voltage or the like, the processor virtually simulates/calculates the electrical field distribution E(x,y,z) that would result in the patient's brain, if the chosen stimulation device 1 would be configured and/or operated with the start parameters (and worn by the patient).

During this simulation, the computer takes into consideration the patient-specific data extracted from the images 3, because the simulation makes use of the 3D-model of the skull calculated from that data. The calculation of the E-field E(x,y,z) is performed by the computer using a state-of-the-art finite element method (FEM); the calculated E-fields E(x,y,z) are also documented in a simulation data file by the computer.

In addition, the computer runs iteratively a series of such simulations, each time varying at least one of said simulation parameters (e.g. virtually changing a certain 3D-location of one of the stimulation electrodes 2, in particular using some random-walk-algorithm) and each time evaluating the calculated electrical field distribution E(x,y,z) using a merit function. In other words, the second step B) is iteratively applied to optimize the generated field distribution E(x,y,z), taking into account the ROI extracted from the pre-existing PET-images 3 with the aim of focusing the simulated field distribution E(x,y,z) in the ROI.

This optimization procedure can be run until no significant progress is made. The resulting set of optimized configuration parameters, which were part of the simulation parameters used in the last simulation, may thus be considered as a "best-possible solution" for a given stimulation device 1.

The whole process may be repeated, starting with a different set of start parameters. For example, a different type of stimulation device 1, featuring a different number of N stimulation electrodes or at least N stimulation electrodes arranged in a different 3d-arrangement, may be chosen by the computer as the starting point of the optimization routine. The single stimulation devices 1 may also be chosen from a pre-defined set of a limited number of M such devices 1, the devices 1 differing from each other for example in the number N and/or specific 3D-position of the stimulation electrodes 2.

Finally, all "best-case" solutions calculated for each of the M devices 1 may be compared to find the stimulation device 1 and associated optimized set of configuration parameters that best fits the needs of the patient, i.e. which would best enable an efficient electrostimulation therapy using one particular stimulation device 1. Of course, such a simulation can also be performed for only one specific type of stimulation device 1.

As shown in Figure 1, the output of the computer-implemented method may thus be the information about which one of the M stimulation devices 1 of the set is best suited and/or which are the best-suited configuration parameters for a particular stimulation device 1, given the skull and brain structure of the patient as described by the images 3.

Based on this output, one of the M stimulation devices 1 (e.g. a bonnet 9 with a size and electrode configuration best suited for the patient) may be chosen (step E) and this particular device 1 may then be configured (step D) using the optimized set of configuration parameters.

As illustrated in Figure 1, a therapy plan, defining a specific electro-therapy scheme to be applied to the patient over a series of electro-therapy sessions (e.g. one session / day, with varying electro-stimulation), may be considered. In fact, the method just explained can be performed repeatedly to calculate a series of individual sets of optimized configuration parameters. Each set can then be used prior to a specific therapy session of the series to configure the stimulation device 1. By applying the method, each set can be optimized (prior to the therapy) for enabling a pre-defined electrostimulation-pattern to be generated with the stimulation device 1 during one of the sessions.

Illustrated in Figure 1 is also that a health care professional (HCP) may provide further input for the simulation to be performed in step B. For example, the HCP may initially chose the stimulation device 1 (e.g. based on medical judgement or primary clinical trials) for which the configuration parameters are to be optimized. The HCP may deliver such information to the computer system by choosing a certain set of start simulation parameters [S₁ , S₂, ... S_{N}].

Moreover, HCPs can also deliver E-field distributions E(x,y,z), for example as recorded with a particular stimulation device 1 configured with a specific set of configuration parameters [p₁ , p₂, ... pᵢ]. Such data can then be compared (by the HCP or the computer itself) with the simulated field distribution and employed for further optimization of the configuration parameters.

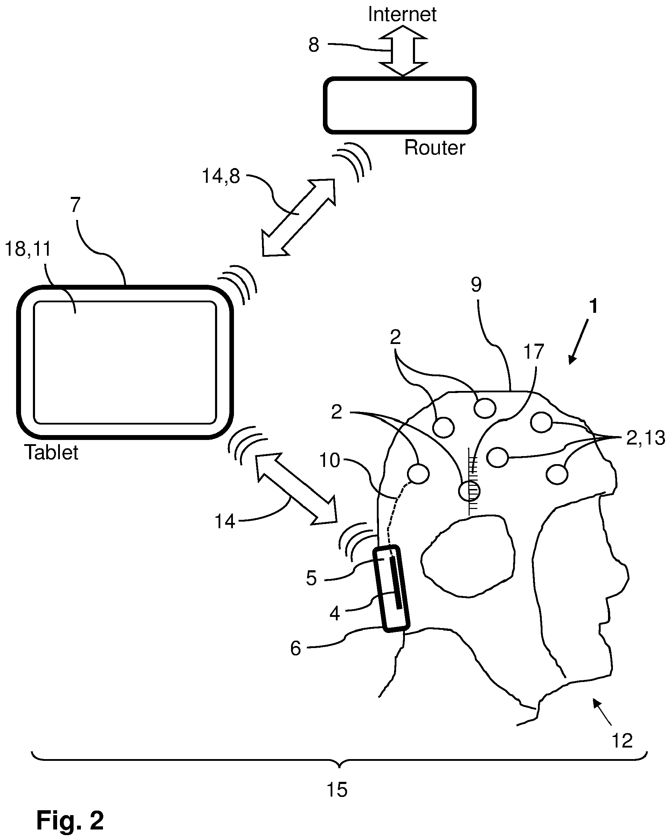

Figure 2 schematically sketches a system 15 for patient-specific electrostimulation according to the invention, comprising an electrical stimulation device 1 according to the invention and a mobile electronic device 7, namely a tablet.

The stimulation device 1 is designed as a bonnet 9 that can be worn by the patient on his head 12. The bonnet 9 carries a number of N non-invasive stimulation electrodes 2 arranged in a 3D-geometry, an electronic driving and read-out circuit 4 that can provide individual electrical driving voltages to the individual electrodes 2 according to an electrical stimulation protocol, and an electrical power supply 6 for autonomous operation of the circuit 4.

As illustrated, some of the electrodes 2 can also be configured electronically as electrical recording electrodes 13 than can be read-out with the electronic circuit 4. Thereby, nerve signals but also E-fields generated with the remaining stimulation electrodes 2 can be recorded with the device 1, when it is worn on the head by the patient.

The device 1 also features a wireless data interface 5 for communication with the tablet 7 via a bidirectional wireless connection 14. The tablet 7 itself is connected to and accessible from the internet via an internet interface 8 that is realized by wirelessly establishing a second connection 14 between the tablet 7 and an internet router.

The data interface 5 of the device 1 is primarily designed to configure the device 1 with a set of optimized configuration parameters derived with the method according to Figure 1. The data interface 5 may be used, however, also for delivering digital data documenting a performed electrostimulation with the device or recorded E-fields and/or nerve signals to the tablet 7. In other words, the data interface 5 of the device 1 is configured to enable configuration and/or control of the stimulation device 1 via the tablet or any other suitable electronic device 7.

Part of the configuration parameters, for examples "best-suited" locations of the individual stimulation electrodes 2 within a certain adjustment range 17, may also be presented to the patient on the display 18 of the tablet 7, such that the patient or a HCP can configure the device 1 accordingly.

One advantage of the approach illustrated in Figure 2 is that is allows remote-control and live-supervision of an electrostimulation therapy performed with the device 1: Through the internet interface 8, a remote-connection to the electronic device 7 can be established and from there to the device 1, in particular for delivering the configuration parameters from a remote computer system or database to the device 1. This remote-connection can also enable an HCP or a computer system to live-monitor, supervise and - in case of danger - to stop an electrostimulation performed with the stimulation device 1.

Another advantage discussed before is that such a system 15 enables tracking of the success of an electrostimulation therapy performed with the device 1 over time, based on input from cognitive tests that the patient has performed using the tablet 7. Via the tablet 7, this input/feedback may be easily delivered to a remote computer system, e.g. of a hospital.

Figure 3 finally illustrates, that the calculation of a set of optimized configuration parameters, and the consequent configuration of a chosen electrical stimulation device 1 may form an important piece of a more general concept of providing patient-specific diagnostic, care and electrostimulation therapy: The hardware and software configuration that is achievable with the methods and devices presented herein, including computer-implemented iterations of the method steps A)-C) for improving this configuration / setup, can form the basis of a complex cycle of regular diagnostic scans (using MRT, PET and the like), experimental data from cognitive tests performed by the patient, data analysis and patient-specific treatment. The method and devices presented herein provide the patient and the HCP with a best-possible stimulation solution for a given design and adaptability of a particular device 1.

In summary, a computer-implemented method is proposed that allows patient-specific choice and/or configuration of an electrical stimulation device 1 that is designed and usable for electrostimulation therapy of neuronal tissue, in particular in the brain. The method benefits from pre-existing images 3 acquired using a medical imaging technique such as MRT or PET. Based on data extracted from such an image 3, a computer simulates possible electrical field distributions E(x,y,z) which would be achievable if the device 1 would be configured with a certain set of configuration parameters. By varying these parameters and repeating the simulations, the method allows optimization of the configuration parameters for a given design of the device 1. As a result, the method delivers a set of optimized configuration parameters, which, if applied to the device 1, allow a "best-case" electrostimulation, tailor-made for the need of the patient, with the device (cf. Figure 1).

### List of reference numerals

- 1: electrical stimulation device
- 2: stimulation electrode (of 1)
- 3: images (e.g. acquired by MRI, CT, PET or like methods)
- 4: electronic driving circuit (of 1)
- 5: data interface (of 1)
- 6: power supply (of 1)
- 7: electronic device
- 8: internet interface (of 7)
- 9: bonnet
- 10: interconnections (between 2 and 4)
- 11: user interface (e.g. touch display, microphone, external keyboard / mouse etc.)
- 12: patient's head
- 13: recording electrode
- 14: wireless connection (bidirectional)
- 15: system for patient-specific electrostimulation
- 16: computer system
- 17: adjustment range (of position of 2)
- 18: display

## Claims

1. **Computer-implemented method** for computing a patient-specific set of configuration parameters suitable for configuring and/or choosing an electrical stimulation device (1) designed for electrical stimulation and/or recording of neuronal tissue, in particular of cerebral tissue, the method comprising the following steps:
A) extracting patient-specific data from at least one pre-existing image (3) previously acquired using a medical imaging technique;
B) computer simulation of an electrical field distribution that can be generated using the electrical stimulation device (1) when configured with a specific set of configuration parameters, taking into consideration the extracted patient-specific data;
C) Variation of a set of simulation parameters during iterative application of step B) to compute the patient-specific set of configuration parameters, which are thus optimized for the patient.

2. Method according to claim 1,
- wherein step C) comprises a variation of a physical configuration of N stimulation electrodes (2) of the stimulation device (1),
- in particular wherein the variation comprises a change of a location of one of the N stimulation electrodes (2) or a size and/or shape of one of the N stimulation electrodes (2), and/or
- wherein N stimulation electrodes (2) of the stimulation device (1) can generate said electrical field distribution.

3. Method according to claim 1 or 2, wherein the configuration parameters comprise at least one, preferably at least two, of the following parameters:
- a specific geometrical arrangement of N stimulation electrodes (2) of the stimulation device (1) achievable by re-arranging the electrodes (2) within a limited geometrical adjustment range offered by the stimulation device (1);
- a specific geometrical arrangement of N stimulation electrodes (2) belonging to one particular stimulation device (1) comprised in a limited number M of stimulation devices (1) each featuring a pre-defined, in particular non-reconfigurable, respective arrangement of the N stimulation electrodes (2);
- a relative distribution of N driving currents or driving voltages to be applied to N stimulation electrodes (2) of the stimulation device (1);

4. Method according to any of the previous claims, wherein the configuration parameters comprise a patient-specific electrical stimulation protocol for driving a number of N stimulation electrodes (2) of the device (1),
- in particular wherein the stimulation protocol comprises at least one, preferably at least two, of the following electrical stimulation parameters:
- amperage and/or
- frequency and/or
- ac/dc driving scheme and/or
- relative phase
of individual driving currents to be applied to the individual stimulation electrodes (2) of the stimulation device (1).

5. Method according to any of the previous claims, wherein the method further comprises a step of
D) configuring the stimulation device (1) with the derived set of configuration parameters, in particular with said electrical stimulation protocol,
- in particular such that the stimulation device (1) is configured to focus the electrostimulation onto at least one region of interest (ROI) that has been extracted from the at least one image (3) during step A).

6. Method according to any of the previous claims, wherein the method further comprises a step of
E) choosing said electrical stimulation device (1) out of a pre-defined set of M different stimulation devices (1) based on the derived set of configuration parameters,
- in particular wherein the M stimulation devices (1) comprised in the pre-defined set differ:
- in their respective number N of available individual stimulation electrodes (2) and/or
- in a respective, fixed or adjustable, geometrical arrangement of a number of N stimulation electrodes (2) and/or
- in a respective individual shape of at least one of the respective stimulation electrodes (2).

7. Method according to any of the previous claims, wherein the patient-specific data extracted in step A) comprise at least one, preferably ate least two, of the following patient-specific parameters:
- geometrical data of a skull of the patient, such as distribution of a thickness of the skull bone and/or of the scalp;
- 3D-distribution of brain tissue, in particular size of the patient's brain and/or distribution of gray and white matter within the brain of the patient;
- localized concentrations of specific proteins, in particular of amyloid and/or tau proteins, within the brain of the patient;
- localized occurrence of atrophy in the brain of the patient;
- 3d-coordinates of at least one region of interest (ROI) to be electro-stimulated with the device (1);
- preferably wherein the optimization performed in step B) takes into account at least one of the these patient-specific parameters for computing said set of configuration parameters, and/or
- wherein the optimization performed in step B) is based on a geometrical head model derived from the patient-specific data extracted in step A).

8. Method according to any of the previous claims, wherein the simulation parameters varied in step C) comprise at least one, preferably at least two, of the following parameters:
- number N of available stimulation electrodes (2) of the device (1);
- geometrical arrangement of N available stimulation electrodes (2) of the device (1);
- respective shape of the available stimulation electrodes (2) of the device (1);
- amperage and/or frequency of driving currents to be applied to individual stimulation electrodes (2) of the device (1);
- electrical ac/dc-driving scheme to be applied to the device (1);

9. Method according to any of the previous claims, wherein step C) is performed
- to maximize a local electrical field strength in at least one region of interest (ROI) extracted from the at least one image in step A), in particular while minimizing the electrical field strength in remaining tissue regions and/or
- using a state-of-the-art optimization algorithm, preferably based on a finite-element-method, and/or
- such that in remaining regions outside of the at least one region of interest (ROI), extracted from the at least one image in step A), an effective field strength remains below a physiological action potential (AP) of neuronal cells present in these remaining regions.

10. Method according to any of the previous claims, wherein during step C) at least one, preferably at least two, of the following boundary conditions are taken into account:
- maximum driving voltage and/or driving current available for driving an individual stimulation electrode (2) of the device (1);
- available frequency range for a driving current to be applied to an individual stimulation electrode (2) of the device (1);
- number M of available fixed geometrical arrangements of N stimulation electrodes (2) provided by a limited set of M available different stimulation devices (1);
- maximum number Nₘₐₓ of available stimulation electrodes (2) ;
- limited number W of different available shapes of stimulation electrodes (2).

11. Method according to any of the previous claims, wherein the at least one pre-existing image (3) comprises at least one of, preferably at least two of,
- a magnetic resonance tomography (MRT) image (3) and/or
- a positron emission tomography (PET) image (3) and/or
- a X-ray computed tomography (CT) image (3).

12. Method according to any of the previous claims, wherein step A) comprises extracting localized concentrations of particular proteins, defining respective regions of interest (ROI), from pre-existing PET-images (3),
- in particular PET-images (3) acquired using protein-specific, in particular radio-active, markers.

13. Method according to of the previous claims, wherein the stimulation device (1) features at least one electrode, in particular one of the N stimulation electrodes (2), configured or configurable for recording
- electrical fields generated with some of the N stimulation electrodes (2) and/or
- nerve signals from neuronal tissue, in particular brain waves,
- preferably wherein, electrical fields and or nerve signals previously recorded with the stimulation device (1) are used as an input for optimizing the configuration parameters during step B) and C).

14. **Method for computing a series of individual sets of optimized configuration parameters,** wherein each set is computed by performing a method according to any of the preceding claims,
- in particular wherein each of the individual sets is intended for configuring the stimulation device (1) prior to a specific therapy session of a series of such sessions, and/or
- wherein each of the sets is optimized for enabling a pre-defined electrostimulation-pattern to be generated with the stimulation device (1),
- preferably wherein the different consecutive electrostimulation-patterns define a specific electro-therapy scheme.

15. **Electrical stimulation device** (1), comprising
- a number of N non-invasive stimulation electrodes (2) arranged in a 3D-geometry,
- an electronic driving circuit (4) for providing individual electrical driving voltages or driving currents to the individual stimulation electrodes (2)
- a data interface (5) for configuring said stimulation device (1) with a patient-specific set of configuration parameters,
- preferably wherein the stimulation device (1) is configured with a set of configuration parameters that have been derived with a method according to one of the previous claims.

16. Electrical stimulation device (1), according to the preceding claim, wherein the stimulation device (1) is designed as a bonnet (9) to be worn by a patient on his skull, the bonnet (9) carrying
- the N stimulation electrodes (2),
- preferably and the driving circuit (4),
- most preferably and an electrical power supply (6) for autonomous operation of the driving circuit (4),
- preferably wherein electrical interconnections (10) between the stimulation electrodes (2) and the driving circuit (4) are embedded in the bonnet (9),
- in particular such that they cannot be manipulated by a patient without destructing the bonnet (9).

17. Electrical stimulation device (1), according to one of the two preceding claims,
- wherein a geometrical arrangement of the N stimulation electrodes (2) is re-configurable or
- wherein a geometrical arrangement of the N stimulation electrodes (2) is permanently or initially fixed and hence not re-configurable, and/or
- wherein the, preferable wireless, data interface (5) is configured to enable configuration and/or control of the stimulation device (1) via an external, preferably mobile, electronic device (7), such as a computer or a tablet.

18. **Set of a limited number M of different electrical stimulation devices (1),** each offering a respective number of N individually addressable non-invasive stimulation electrodes (2),
- in particular wherein each of said stimulation devices (1) is a stimulation device (1) according to one of the three preceding claims, **characterized in that**
- the stimulation devices (1) comprised in the set differ in terms of
- their respective number N of available individual stimulation electrodes (2) and/or
- a respective fixed geometrical arrangement of a number of N stimulation electrodes (2) and/or
- a respective individual shape of at least one of the respective stimulation electrodes (2).
- preferably wherein the number M is smaller than 10.

19. **System (15) for patient-specific electrostimulation** of neuronal tissue, comprising
- an electrical stimulation device (1), in particular according to one of the claims 12 - 14, and
- a, preferably mobile, electronic device (7), which can communicate with and control the electrical stimulation device (1) via a, preferably wireless, data interface (5) of the stimulation device (1),
**characterized in that**,
- the electronic device (7) is configured to transmit a set of configuration parameters,
- in particular derived using a method according to one of the claims 1 to 11,
to the stimulation device (1) via the data interface (5) of the stimulation device (1) and/or
- the electronic device (7) features an internet interface (8) for remote-control of the electronic device (7) and thereby of the stimulation device (1),
- preferably such that an electrostimulation performed with the stimulation device (1) can be live-monitored and/or stopped via a remote-connection to the electronic device (7) established using its internet interface (8).

20. **Computer program,** preferably designed as an app to be run on a mobile electronic device (7), comprising instructions, which, when the program is executed by a computer/said electronic device (7), cause the computer/said electronic device (7) to carry out a method according to one of the claims 1-11.

21. **Computer program,** preferably designed as an app to be run on a mobile electronic device (7), comprising instructions, which when carried out by an external mobile electronic device (7) or a computer, cause the computer / electronic device (7)
- to communicate with an electrical stimulation device (1), in particular according to one of the claims 12 to 14, and
- to configure the stimulation device (1) with a set of configuration parameters enabling patient-specific electrostimulation,
- preferably wherein the set of configuration parameters has been derived with a method according to one of the claims 1-11,
and/or
- to establish a remote-connection to the electronic device (7) via the internet thus enabling live-monitoring of and/or stopping an electrostimulation performed with the electrical stimulation device (1).

22. **Computer program,** according to the previous claim, wherein the program comprises instructions, which, if carried out, by the electronic device (7) cause the electronic device (7)
- to offer the user a cognitive test, either before or after an electrostimulation has been performed with the electrical stimulation device (1), in particular using a display and our loudspeakers and/or a microphone of the electronic device (7),
- in particular and to record nerve signals from the patient using a recording electrode (13) of the stimulation device (1) while the patient performs the cognitive test, and
- to digitally document a bio marker, for example a success rate of a cognitive test result, based on a response to that cognitive test, which the user has input into the electronic device (7) via a user interface (11),
- in particular such that the electronic device (7) records a time stamp and or parameters of a performed electrostimulation together with the bio marker.
